# EUROPEAN PATENT SPECIFICATION

(11) **EP 0 329 062 B1**
(45) Date of publication and mention of the grant of the patent: **18.05.1994**
(21) Application number: 89102479.6
(22) Date of filing: 14.02.1989
(51) Int. Cl.: C12N 1/21, C12P 17/10, C12P 19/38

(54) **A pyrimidine analogue resistance gene DNA and its use**
Ein DNS-Resistenzgen gegen Pyrimidine Analoge und dessen Verwendung.
Un gène d'ADN de résistance à des analogues de pyrimidine et son utilisation

(30) Priority: 17.02.1988 JP 36398/88
(43) Date of publication of application: 23.08.1989
(73) Proprietor: Takeda Chemical Industries, Ltd., Chuo-ku, Osaka (JP)
(72) Inventor: Asahi, Satoru, Suita Osaka 565 (JP); Tsunemi, Yutaka, Suita Osaka 565 (JP); Doi, Muneharu, Takarazuka Hyogo 665 (JP)
(74) Representative: von Kreisler, Alek, Dipl.-Chem.

(56) References cited:
- EP-A- 0 080 378
- EP-A- 0 179 468
- MGG Molecular and General Genectics, vol. 144, no. 3, 1976, pp. 313-324, Berlin, DE; E. LOVE et al.: "Mapping of the gene specifying DNA Polymerase III of bacillus-subtilis"
- Agricultural and Biological Chemistry, vol. 50, no. 1, January 1986, pp. 9-15, Tokyo, JP; M. Shimosaka et al.: "Application of a Plasmid Carrying a Gene for Orotate, Phosphoribosyltransferase (pyrE) to Orotidine-5'-monophosphate Production in Eschericia coli K-12"
- MGG Molecular and General Genetics vol. 205, no. 1, 1986, pp. 74-81, Berlin, DE; P. LILJELUND et al.: "Genetic characterization and isolation of the saccharomyces-cerevisiae gene coding for uridine monophosphokinase"
- BIOCHEMICAL GENETICS, vol. 18, no. 78, 1980, pp. 717-726, New York, US; O'BYRNE et al.: "Biochemical and genetic basis of the response to 5 fluoro uracil in drosophila-melanogaster"
- CHEMICAL ABSTRACTS, vol. 96, no. 21, 24th May 1982, p. 164, column 1, abstract no. 175212e, Columbus, Ohio, US; K.F. JENSEN et al.: "RNA polymeraseinvolvement in the regulation of expression of Salmonella typhimurium pyr genes.Isolation and characterization of a fluorouracil-resistant mutant with high, constitutive expression of the pyrB and pyrE genes due to a mutation in rpoBC" & EMBO Journal, vol. 1, no. 1, 1982, pp. 69-74

## Description

### FIELD OF THE INVENTION

The present invention relates to a DNA containing a pyrimidine analogue resistance gene, a plasmid incorporating the DNA, microorganisms of the genera Escherichia and Bacillus transformed with the plasmid, and a method for producing uracil and/or uridine by utilizing the microorganisms.

### BACKGROUND OF THE INVENTION

Uracil and uridine both serve well as starting materials for synthesis of biochemical reagents or pharmaceuticals. However, wild bacterial strains produce little uracil and/or uridine extracellularly and, therefore, in order to produce uracil and/or uridine by fermentation, there is used the method in which artificial mutation is induced on a wild strain to provide it with capability of producing uracil and/or uridine. Conventional fermentative production methods include the method using a pyrimidine analogue-resistant strain derived from a microorganism of the genus Micromonospora (Japanese Patent Publication No. 18873/1982), the method using a purine analogue resistant strain derived from a bacterium of the genus Brevibacterium (Japanese Patent Publication No. 30476/1982), and the method using a strain lacking uridine nucleoside phosphorylase activity and possessing pyrimidine analogue resistance derived from a microorganism of the genus Bacillus (European Patent Application-A2-0 179 468).

With regard to the improvement of uracil and/or uridine producers, the above-described methods are already known. However, indeed, none of the conventional methods of mutagenic treatment permits an increase in yield by selective amplification of a specific gene.

### OBJECTS OF THE INVENTION

The present inventors have conducted intensive research with the aim of utilizing gene recombination technology to develop uracil producing microorganisms, and they have found that a strain obtained by transforming a microorganism of the genus Escherichia or Bacillus with a plasmid incorporating a pyrimidine analogue resistance gene of Bacillus subtilis AA 47 (IFO 14705, FERM BP 2183) produces an improved yield of uracil and uridine in the media. The present inventors have made further investigations based on this finding and attained the present invention.

That is, one object of the present invention is to provide uracil producing microorganisms which can produce these materials in an improved yield.

Another object of the present invention is to provide a method for producing uracil by using these microorganisms.

These objects as well as other objects and advantages of the present invention will be apparent to those skilled in the art from the following description with reference to the accompanying drawing.

### BRIEF EXPLANATION OF DRAWING

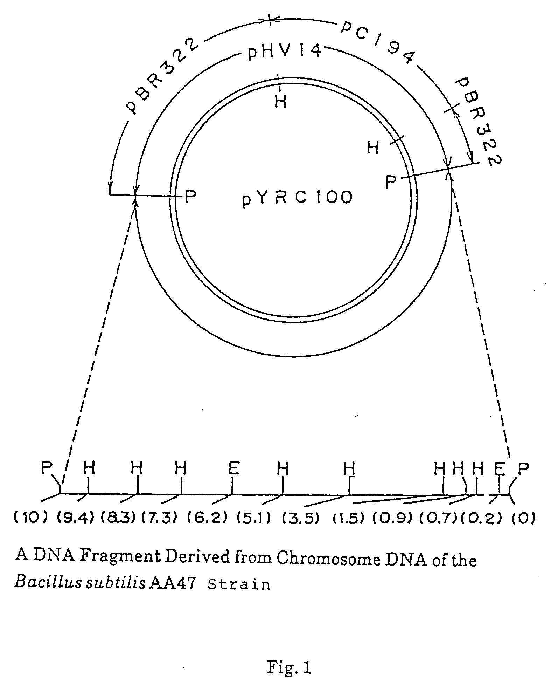

Fig. 1 is a restriction enzyme map of the recombinant plasmid pYRC100 as obtained in Example 1 hereinafter. The figures in parentheses represent the number of kilobase pairs (kb). H represents a HindIII cleavage site, P represents a PstI cleavage site, and E represents an EcoRI cleavage site.

### SUMMARY OF THE INVENTION

According to the present invention, there are provided the following DNA, plasmid incorporating the DNA, microorganisms transformed with the plasmid, and method for producing uracil and/or uridine by using the microorganism.
(1) A DNA having the pyrimidine analogue resistance gene as defined in fig 1 which has PstI cleavage points about 10 kb apart and two EcoRI restriction sites and eight HindIII restriction sites in the PstI fragment, and which is obtainable from the chromosomal DNA of Bacillus subtilis AA47 (IFO 14705, FERM BP 2183) which is resistant to pyrimidine analogues and capable of producing uracil and uridine.
(2) A plasmid incorporating the DNA as defined in the above item (1).
(3) A pure culture of a microorganism of the genus Escherichia or Bacillus transformed with the plasmid as defined in the above item (2).
(6) A method for producing uracil which comprises cultivating in a medium a microorganism of the genus Escherichia or Bacillus which is transformed with a plasmid (above item (2)) incorporating the pyrimidine analogue resistance gene obtained from the chromosomal DNA of Bacillus subtilis (IFO 14705, FERM BP 2183) resistant to pyrimidine analogues, to thereby cause formation and accumulation of uracil in the culture broth, and recovering the uracil from said broth.

### DETAILED DESCRIPTION OF THE PRESENT INVENTION

The DNA of the present invention is obtained from microbes resistant to pyrimidine analogues.

In the present specification, the term "pyrimidine analogue-resistant strains" means microorganisms which are derived from a bacterium of the genus Escherichia or Bacillus as the parent strain and which have changed in genetic characters so that they are capable of growing in media containing one or more pyrimidine analogues in such a concentration that it is too high for the parent strain to grow therein. And, the term "pyrimidine analogues" means substances having a structure similar to that of pyrimidine bases, such as uracil and cytosine, e.g. 6-azauracil, 2-thiouracil, 5-fluorouracil, 2-thiocytosine, 5-fluorocytosine, and ribosides and ribotides thereof. Any pyrimidine analogue-resistant strain can serve for the present invention so far as it is resistant to at least one pyrimidine analogue.

The DNA donor is Bacillus subtilis AA47 (IFO 14705, FERM BP 2183).

Examples of methods for preparing a DNA fragment containing the pyrimidine analogue resistance gene include the method in which chromosomal DNA is extracted from the donor by the method of Saito and Miura [H. Saito and K. Miura, Biochimica et Biophysica Acta, 72, 619 (1963)] and then cleaved using the restriction enzyme PstI. The chromosomal DNA fragment containing the pyrimidine analogue resistance gene thus obtained is then inserted into a vector DNA.

Vector DNA's used for the present invention can be appropriately chosen from vectors capable of proliferating in bacteria of the genus Escherichia, or those capable of proliferating in bacteria of the genus Bacillus, or those capable of proliferating in bacteria of the two genera (so-called shuttle vectors). Examples of plasmids capable of proliferating in bacteria of the genus Escherichia include pSC101 [Proceedings of the National Academy of Science, USA, 70, 3240 (1973)], pBR322 [Gene, 2, 95 (1977)] and pBR325 [Gene, 4, 121 (1978)]. Examples of plasmids capable of proliferating in bacteria of the genus Bacillus include pUB110 [Journal of Bacteriology, 134, 318 (1978)], pC194 [Proceedings of the National Academy of Science, USA, 74, 1680 (1977)] and pLS28 [Journal of Bacteriology, 131, 699 (1977)]. Examples of shuttle vectors capable of proliferating in bacteria of both genera Escherichia and Bacillus include pHV14 [Proceedings of the National Academy of Science, USA, 75, 1433 (1978)] and pDH5060 [Molecular and General Genetics, 193, 299 (1984)]. In addition to these known plasmids, vector DNA's which have recently been isolated or synthesized can also be used so far as they can serve for the present invention.

For inserting a DNA fragment containing the pyrimidine analogue resistance gene into these plasmid vectors, there can be used, for example, the methods described by T. Maniatis et al. in "Molecular Cloning, A Laboratory Manual", Cold Spring Harbor Laboratory, University of Tokyo Press (1982).

For introducing a plasmid vector incorporating the pyrimidine analogue resistance gene into a recipient, there can be used ordinary methods of transformation such as, for bacteria of the genus Escherichia, the method comprising calcium chloride treatment of recipient cells and subsequent DNA introduction [Journal of Molecular Biology, 53, 159 (1979)] and, for cells of bacteria of the genus Bacillus, the method comprising protoplast preparation from recipient and subsequent DNA introduction [Molecular and General Genetics, 168, 111 (1979)] and the method using competent cells [Journal of Bacteriology, 81, 714 (1961)]. Bacteria which can be used as recipients include Escherichia coli, Bacillus subtilis, Bacillus pumilus, and Bacillus licheniformis and it is irrespective whether or not the recipient is capable of producing uracil and/or uridine. Examples of strains of such recipients include known strains such as Escherichia coli C600 [Bacteriology Review, 36, 525 (1972)], Bacillus subtilis (IFO 13719, ATCC 6051) and the like. Where the vector DNA used has a selection marker for antibiotic resistance etc., the selection of the desired transformant strain can be further facilitated, for instance, by the addition of the relevant antibiotic to the above-described selection medium. The vector DNA incorporating the pyrimidine analogue resistance gene thus obtained can be used in ligation to other vector plasmids after extraction from the donor strain and introduction to other recipients, or after preparation of a DNA fragment containing the pyrimidine analogue resistance gene from the recombinant DNA thus extracted. Examples of transformant strains thus obtained include Escherichia coli TFA17 (IFO 14704, FERM P-9865) which is a transformant strain of Escherichia coli C600, Bacillus subtilis TFBA16 (IFO 14707, FERM P-9867, FERM BP-2184) which is a transformant strain of Bacillus subtilis (IFO 13719, ATCC 6051), and Bacillus subtilis TFAA47 (IFO 14708, FERM P-9868) which is a transformant strain of Bacillus subtilis AA47 (IFO 14705, FERM P-9866).

The above-described strain Bacillus subtilis AA47 has been derived from the known strain, Bacillus subtilis (IFO 13719, ATCC 6051) and is resistant to pyrimidine analogues and capable of producing uracil and uridine. However, its other bacteriological characteristics are the same as those of its parent strain.

In the present specification, the IFO numbers represent accession numbers at the Institute for Fermentation, Osaka (IFO, 17-85, Juso-honmachi 2-chome, Yodogawa-ku, Osaka-shi, Japan) and the FERM P numbers represent accession numbers at the Fermentation Research Institute, Agency of Industrial Science and Technology (FRI, 1-3, Higashi 1-chome, Tsukuba-shi, Ibaraki-ken, Japan). The strains under the accession numbers IFO 14704, IFO 14705, IFO 14707 and IFO 14708 described in the present specification have been deposited at IFO since January 19, 1988, and the strains under FERM P-9865, FERM P-9866, FERM P-9867 and FERM P-9868 deposited at FRI since February 10, 1988. The deposits in FRI have been converted into those under Budapest Treaty as of December 8, 1988 and the following FERM-BP numbers have been assigned.

| FERM P- No. | FERM BP- No. |
|---|---|
| 9865 | 2182 |
| 9866 | 2183 |
| 9867 | 2184 |
| 9868 | 2185 |

For cultivating the uracil producing microorganisms obtained in the above, it is possible to use microbial cultivation methods similar to those which have conventionally been used to produce this substance. That is, there can be used media containing carbon sources, nitrogen sources, metal ions and, as needed, amino acids, nucleic acids, vitamins and other nutrients. Examples of carbon sources include glucose, sucrose, maltose, starch, saccharified starch solutions and molasses. Examples of nitrogen sources include organic nitrogenous substances such as peptone, corn steep liquor, soybean meal, yeast and urea; and inorganic nitrogenous substances such as ammonium salts of sulfuric acid, nitric acid, hydrochloric acid, carbonic acid, etc., gaseous ammonia and aqueous ammonia. These substances can be used alone or in combination thereof. As other nutrients, various inorganic salts, amino acids, vitamins, etc. which are essential to the growth of the bacteria are chosen appropriately and they are used alone or in combination. Furthermore, defoaming agents and surfactants such as silicon oil and polyalkylene glycol ether can also be added to the medium when necessary. Cultivation is normally conducted under aerobic conditions, for instance, by shaking culture or aerobic deep submerged culture. Normally, the pH of media in the range of between 4 and 9 is preferred. If a pH varies during cultivation, sulfuric acid, calcium carbonate, sodium hydroxide, gaseous ammonia, aqueous ammonia etc. can be added appropriately to adjust the pH to a level in the desired range. The cultivation temperature is normally chosen in the range of 20°C to 45°C in view of the desired growth of the microorganism used and the desired uracil accumulation. Cultivation is continued until the amount of uracil accumulated substantially reaches the maximum. Normally, this purpose can be attained by cultivation for 24 to 144 hours.

For separating and collecting uracil from the culture, there can be used conventional separation and purification methods, for example, precipitation and chromatography using an ion exchange resin or activated charcoal.

The following Examples further illustrate the present invention in detail but should not be construed to limit the scope thereof.

### Example 1

### i) Preparation of chromosomal DNA

Bacillus subtilis AA47 strain (IFO 14705, FERM BP-2183), capable of producing uracil and/or uridine and resistant to pyrimidine analogues, was inoculated into 1 liter of L-medium (1.0% of Bacto-trypton, 0.5% of yeast extract, 0.5% of sodium chloride) and cultivated at 37°C overnight. The bacterial cells thus obtained were subjected to the method of Saito et al. [Biochimica et Biophysica Acta, 72, 619 (1963)] to obtain, finally, 6.0 mg of a chromosomal DNA.

### ii) Insertion of chromosomal DNA into the vector plasmid pHV14

The following experimental procedures were conducted according to the method described by T. Maniatis et al. in "Molecular Cloning, A Laboratory Manual", Cold Spring Harbor Laboratory, Tokyo University Press (1982), unless otherwise stated.

10 µg of the chromosomal DNA obtained in the above item i) and 10 µg of pHV14 (manufactured by Nippon Gene, Japan) were cleaved with the restriction enzyme PstI (manufactured by Nippon Gene, Japan), respectively. Then, they were mixed together and subjected to ligation using T4 phage-derived DNA ligase (manufactured by Nippon Gene, Japan).

### iii) Cloning of pyrimidine analogue resistance gene

The competent cell method was used for transformation. Namely, the plasmid DNA prepared in the above item ii) was added to a competent cell suspension prepared from Escherichia coli C600 strain and incorporated into the competent cells for transformation. This suspension containing the transformed cells was then spread over a plate of MAC-9 agar medium (0.6% of sodium dihydrogen phosphate, 0.3% of potassium dihydrogen phosphate, 0.1% of ammonium chloride, 0.05% of sodium chloride, 1 mM of magnesium sulfate, 1mM of calcium chloride, 0.3% of Casamino acids, 0.01% of L-tryptophan, 0.5% of glucose and 2.0% of agar; pH 7.2) supplemented with 200 µg/ml of 6-azauracil and 10 µg/ml of chloramphenicol and cultivated at 37°C for two days. From the colonies that formed on the plate medium, a transformant resistant to both chloramphenicol and 6-azauracil, i.e., Escherichia coli TFA17 strain (IFO 14704, FERM BP-2182) was obtained.

### iv) Plasmid extraction from transformant

Finally, 500 µg of a plasmid was obtained from 1 liter of culture broth of the transformant strain Escherichia coli TFA 17 (IFO 14704, FERM BP-2182). This plasmid was named as pYRC100.

### v) Analysis of pYRC100

pYRC100 was cleaved with various restriction enzymes and subjected to agarose gel electrophoresis. The restriction enzyme cleavage map as shown in Fig. 1 was obtained from the agarose gel electrophoresis pattern based on the molecular weight cf the HindIII digestion products of lambda phage DNA (manufactured by Nippon Gene, Japan). pYRC100 is a recombinant DNA having an approximately 10 kb DNA fragment inserted in the PstI site of pHV14 and has EcoRI sites at positions 0.2 and 6.2 kb apart from the Pst I site in the right side of the drawing and, in all, eight HindIII sites at positions 0.7, 0.9, 1.5, 3.5, 5.1, 7.3, 8.3, and 9.4 kb apart from the same PstI site in the right.

### vi) Re-transformation

Escherichia coli C600 was re-transformed with the above-described plasmid DNA in order to confirm the presence of pyrimidine analogue resistance gene on pYRC100. Ten cells were collected from each of the resulting chloramphenicol-resistant colonies and subjected to test for pyrimidine analogue resistance. All the strains tested had acquired pyrimidine resistance. The result indicates that pYRC100 carried the pyrimidine analogue resistance gene. Table 1 shows the growth of Escherichia coli C600 and one of the transformants, Escherichia coli TFA17, on plates of MAC-9 medium containing a pyrimidine analogue or chloramphenicol.

**Table 1**

| Substance added to MAC-9 | Growth of each strain* | |
|---|---|---|
| Figures in parentheses represent amount of substance added (µg/ml) | C600 strain | TFA17 strain |
| Without addition | + | + |
| Chloramphenicol (10) | - | + |
| 6-Azauracil (200) | - | + |
| 5-Fluorouracil (5) | - | + |
| 2-Thiouracil (100) | - | + |
| 5-Fluorocytosine (5) | - | + |
| 5-Fluorocytidine (10) | - | + |

| | | |
|---|---|---|
| * +: Growth occured. -: Growth did not occur. | | |

### vii) Productivity of transformant for uracil and/or uridine.

The above-described transformant, Escherichia coli TFA17, was inoculated into a 200 ml flask containing 20 ml of a fermentation medium composed of 3% of glucose, 0.2% of ammonium sulfate, 0.1% of potassium primary phosphate, 1% of corn steep liquor, 0.01% of magnesium sulfate and 2% of calcium carbonate and subjected to shaking culture at 28°C for 3 days. As the result, 750 mg/l of uracil was accumulated. On the other hand, no uracil accumulation occurred when Escherichia coli C600 was cultivated under the same conditions as described above.

### Example 2

### i) Transformation of Bacillus subtilis with pYRC100.

Using the plasmid pYRC100 as obtained in Example 1 iv), Bacillus subtilis (IFO 13719, ATCC 6051) was transformed by the method in which polyethylene glycol was reacted on recipient protoplast to incorporate DNA [S. Chang and S.N. Cohen; Molecular and General Genetics, 168, 111 (1979)]. The transformants which had grown on the above-described regeneration medium containing 10 µg/ml of chloramphenicol were replicated onto an agar plate of MAC-9 medium containing 10 µg/ml of chloramphenicol and 200 µg/ml of 6-azauracil, and cultivated at 37°C for 2 days. From the resulting colonies, Bacillus subtilis TFBA16 (IFO 14707, FERM BP-2184) was picked. The Bacillus subtilis TFBA16 strain was resistant to both chloramphenicol and pyrimidine analogues. Table 2 shows the growth of the Bacillus subtilis (IFO 13719, ATCC 6051) and Bacillus subtilis TFBA16 strains on an agar plate of MAC-9 medium containing chloramphenicol or a pyrimidine analogue.

**Table 2**

| Substance added to MAC-9 | Growth of each Strain* | |
|---|---|---|
| Figures in parentheses represent amount of substance added (µg/ml) | IFO 13719 | TFBA16 |
| Without addition | + | + |
| Chloramphenicol (10) | - | + |
| 6-Azauracil (200) | - | + |
| 5-Fluorouracil (5) | - | + |
| 2-Thiouracil (100) | - | + |
| 5-Fluorocytosine (5) | - | + |
| 5-Fluorocytidine (10) | - | + |

| | | |
|---|---|---|
| * +: Growth occured. -: Growth did not occur. | | |

### ii) Productivity of transformants for uracil and/or uridine

Bacillus subtilis (IFO 13719, ATCC 6051), Bacillus subtilis TFBA16 and Bacillus subtilis AA47 were inoculated into a 200 ml flask containing 20 ml of a fermentation medium composed of 15% of glucose, 3% of corn steep liquor, 1% of urea and 2% of calcium carbonate, respectively, and subjected to shaking culture at 37°C for 3 days. The results obtained were shown in Table 3.

**Table 3**

| Bacterial strain | Amount of uracil accumulated* | Amount of uridine accumulated* |
|---|---|---|
| Bacillussubtilis (IFO 13719, ATCC 6051) | 0 | 0 |
| Bacillussubtilis (TFBA16) | 2.0 | 0.7 |
| Bacillussubtilis AA47 | 1.6 | 0.5 |

| | | |
|---|---|---|
| *: mg/ml | | |

### Example 3

Using the plasmid pYRC100 according to the same manner as described in Example 2, the Bacillus subtilis AA47 strain (IFO 14705, FERM P-9866, FERM BP-2183) was transformed. The transformants which had grown on a regeneration medium containing 10 µg/ml of chloramphenicol were replicated onto an agar plate of MAC-9 medium containing 10 µg/ml of chloramphenicol and 200 µg/ml of 6-azauracil, and cultivated at 37°C for 2 days. From the resulting colonies, the Bacillus subtilis TFAA47 strain (IFO 14708, FERM BP-2185) was obtained. Table 4 shows the amounts of uracil and uridine produced by inoculating Bacillus subtilis AA and Bacillus subtilis TFAA47 strains into the fermentation medium as described in Example 2 ii) and subjecting them to shaking culture at 37°C for 3 days.

**Table 4**

| Bacterial Strain | Amount of uracil accumulated* | Amount of uridine accumulated* |
|---|---|---|
| Bacillussubtilis AA47 | 1.6 | 0.5 |
| Bacillussubtilis TFAA47 | 3.2 | 1.5 |

| | | |
|---|---|---|
| *: mg/ml | | |

### Example 4

Using fifty 200-ml flasks each containing 20 ml of the fermentation medium as described in Example 2 ii), Bacillus subtilis TFAA47 was cultivated according to the same manner as described in Example 3. Each culture thus obtained was centrifuged to remove bacterial cells. The resulting supernatant was adjusted to pH 2.0 with a 1N hydrochloric acid solution and then centrifuged to remove the precipitate. The uracil and uridine in the supernatant thus obtained were adsorbed to a column of activated charcoal and then eluted with a 50% ethanol solution containing 1.4% aqueous ammonia. The uridine and uracil fractions were collected and concentrated under reduced pressure. The resulting concentrate was adjusted to pH 8.0 by the addition of aqueous ammonia. To this concentrate was added an equal volume of a 0.01 M potassium borate solution, and uracil and uridine were adsorbed with a column of Dowex-lX2 (Cl⁻ type, 200 to 400 mesh). After washing this column with distilled water, uracil and uridine were eluted with an aqueous solution containing 0.08 M potassium chloride and 0.02 M potassium borate per liter. The uracil and uridine fractions were collected independently. An equal volume of methanol was added to each eluate and the mixture was concentrated to dryness. This was repeated. Each solid material thus obtained was dissolved in a small amount of water and to the solutions was added ethyl alcohol and chilled to give 2.0 g of crystals of uracil and 0.8 g of crystals of uridine.

As described hereinabove, according to the present invention, pyrimidine analogue resistance can efficiently be provided to various microorganisms. As the result, even a strain which can not produce uracil and/or uridine can be transformed into uracil and uridine producers by introducing a recombinant DNA containing the pyrimidine analogue resistance gene of the invention into the microorganism. Further, in the case of uracil producer strains, their productivity for this substance can be improved by transforming them according to the method of the present invention.

## Claims (Claims for the following Contracting State(s): AT, BE, CH, DE, FR, GB, GR, IT, LI, LU, NL, SE)

1. A DNA having a pyrimidine analogue resistance gene region wherein said DNA has a restriction enzyme map of Fig. 1 and is obtainable from a chromosomal DNA of a microorganism of Bacillus subtilis AA47 (IFO 14705, FERM BP 2183)

2. A plasmid incorporating the DNA as claimed in claim 1.

3. A pure culture of a microorganism of the genus Escherichia or Bacillus transformed with the plasmid as claimed in claim 2.

4. The pure culture as claimed in claim 3 which is Escherichia coli TFA17 (IFO 14704, FERM BP-2182).

5. The pure culture as claimed in claim 3 which is Bacillus subtilis TFBA16 (IFO 14707, FERM BP-2184).

6. The pure culture as claimed in claim 3 which is Bacillus subtilis TFAA47 (IFO 14708, FERM BP-2185).

7. A method for producing uracil which comprises cultivating in a suitable culture medium a microorganism of the genus Escherichia or Bacillus which is transformed with a plasmid incorporating a pyrimidine analogue resistance gene having a restriction enzyme map of Fig. 1 and obtainable from a chromosomal DNA of a microorganism of Bacillus subtilis AA47 (IFO 14705, FERM BP 2183) to thereby cause formation and accumulation of uracil in the culture broth, and recovering the uracil from said broth.

8. The method as claimed in claim 7, wherein the transformed microorganism is Escherichia coli TFA17 (IFO 14704, FERM BP-2182).

9. The method as claimed in claim 7, wherein the transformed microorganism is Bacillus subtilis TFBA16 (IFO 14707, FERM BP-2184).

10. The method as claimed in claim 7, wherein the transformed microorganism is Bacillus subtilis TFAA47 (IFO 14708, FERM BP-2185).

## Claims (Claims for the following Contracting State(s): ES)

1. A process for preparing a DNA having a pyrimidine analogue resistance gene region wherein said DNA has a restriction enzyme map of Fig. 1 and is obtainable from a chromosomal DNA of a microorganism of Bacillus subtilis AA47 (IFO 14705, FERM BP 2183), which process comprises extraction of the chromosome DNA of the donor microorganisms.

2. A processs for preparing a plasmid incorporating the DNA as prepared in accordance with claim 1, which process comprises inserting the above DNA into a vector DNA.

3. A process for preparing a pure culture of a microorganism of the genus Escherichia or Bacillus capable of producing uracil, which process comprises transforming the recipient microorganism with the plasmid as prepared in accordance with claim 2.

4. The process as claimed in claim 3, wherein the prepared pure culture of the microorganism is Escherichia coli TFA17 (IFO 14704, FERM BP-2182).

5. The process as claimed in claim 3, wherein the prepared pure culture of the microorganism is Bacillus subtilis TFBA16 (IFO 14707, FERM BP-2184).

6. The process as claimed in claim 3, wherein the prepared pure culture of the microorganism is Bacillus subtilis TFAA47 (IFO 14708, FERM BP-2185).

7. A method for producing uracil which comprises cultivating in a suitable culture medium a microorganism of the genus Escherichia or Bacillus which is transformed with a plasmid incorporating a pyrimidine analogue resistance gene having a restriction enzyme map of Fig. 1 and obtainable from a chromosomal DNA of a microorganism of Bacillus subtilis AA47 (IFO 14705, FERM BP 2183) to thereby cause formation and accumulation of uracil in the culture broth, and recovering the uracil from said broth.

8. The method as claimed in claim 7, wherein the transformed microorganism is Escherichia coli TFA17 (IFO 14704, FERM BP-2182).

9. The method as claimed in claim 7, wherein the transformed microorganism is Bacillus subtilis TFBA16 (IFO 14707, FERM BP-2184).

10. The method as claimed in claim 7, wherein the transformed microorganism is Bacillus subtilis TFAA47 (IFO 14708, FERM BP-2185).

## Patentansprüche (Patentansprüche für folgende(n) Vertragsstaat(en): AT, BE, CH, DE, FR, GB, GR, IT, LI, LU, NL, SE)

1. DNA, die eine Genregion der Resistenz gegenüber einem Pyrimidinanalogen besitzt, wobei die DNA eine Restriktionsenzymkarte von Fig. 1 besitzt und aus Chromosomen-DNA des Mikroorganismus *Bacillus subtilis* AA47 (IFO 14705, FERM BP 2183) erhalten wird.

2. Plasmid, das die in Anspruch 1 beanspruchte DNA enthält.

3. Reinkultur eines Mikroorganismus der Gattung *Escherichia* oder *Bacillus*, der mit dem in Anspruch 2 beanspruchten Plasmid transformiert ist.

4. Reinkultur wie in Anspruch 3 beansprucht, welche *Escherichia coli* TFA17 (IFO 14704, FERM BP-2182) ist.

5. Reinkultur wie in Anspruch 3 beansprucht, welche *Bacillus subtilis* TFBA16 (IFO 14707, FERM BP-2184) ist.

6. Reinkultur wie in Anspruch 3 beansprucht, welche *Bacillus subtilis* TFAA47 (IFO 14708, FERM BP-2185) ist.

7. Verfahren zum Herstellen von Uracil, welches das Kultivieren in einem geeigneten Kulturmedium eines Mikroorganismus der Gattung *Escherichia* oder *Bacillus*, der mit einem Plasmid transformiert ist, das ein Gen mit einer Resistenz gegenüber einem Pyrimidinanalogen mit einer Restriktionsenzymkarte von Fig. 1 enthält und das aus Chromosomen-DNA des Mikroorganismus *Bacillus subtilis* AA47 (IFO 14705, FERM BP 2183) erhalten wird, um dadurch die Bildung und Anreicherung von Uracil in der Kulturbrühe zu bewirken, und das Gewinnen des Uracils aus der Kulturbrühe umfaßt.

8. Verfahren wie in Anspruch 7 beansprucht, bei welchem der transformierte Mikroorganismus *Escherichia coli* TFA17 (IFO 14704, FERM BP-2182) ist.

9. Verfahren wie in Anspruch 7 beansprucht, bei welchem der transformierte Mikroorganismus *Bacillus subtilis* TFBA16 (IFO 14707, FERM BP-2184) ist.

10. Verfahren wie in Anspruch 7 beansprucht, bei welchem der transformierte Mikroorganismus *Bacillus subtilis* TFAA47 (IFO 14708, FERM BP-2185) ist.

## Patentansprüche (Patentansprüche für folgende(n) Vertragsstaat(en): ES)

1. Verfahren zum Herstellen einer DNA, die eine Genregion der Resistenz gegenüber einem Pyrimidinanalogen besitzt, wobei die DNA eine Restriktionsenzymkarte von Fig. 1 besitzt und aus Chromosomen-DNA des Mikroorganismus *Bacillus subtilis* AA47 (IFO 14705, FERM BP 2183) erhalten wird, welches die Extraktion der Chromosomen-DNA aus den Donor-Mikroorganismen umfaßt.

2. Verfahren zum Herstellen eines Plasmids, das die gemäß Anspruch 1 hergestellte DNA enthält, welches das Einführen der vorstehenden DNA in eine Vektor-DNA umfaßt.

3. Verfahren zum Herstellen einer Reinkultur eines zum Produzieren von Uracil befähigten Mikroorganismus der Gattung *Escherichia* oder *Bacillus*, welches das Transformieren des Empfängermikroorganismus mit dem gemäß Anspruch 2 hergestellten Plasmid umfaßt.

4. Verfahren wie in Anspruch 3 beansprucht, bei welchem der Mikroorganismus der hergestellten Reinkultur *Escherichia coli* TFA17 (IFO 14704, FERM BP-2182) ist.

5. Verfahren wie in Anspruch 3 beansprucht, bei welchem der Mikroorganismus der hergestellten Reinkultur *Bacillus subtilis* TFBA16 (IFO 14707, FERM BP-2184) ist.

6. Verfahren wie in Anspruch 3 beansprucht, bei welchem der Mikroorganismus der hergestellten Reinkultur *Bacillus subtilis* TFAA47 (IFO 14708, FERM BP-2185) ist.

7. Verfahren zum Herstellen von Uracil, welches das Kultivieren in einem geeigneten Kulturmedium eines Mikroorganismus der Gattung *Escherichia* oder *Bacillus*, der mit einem Plasmid transformiert ist, das ein Gen mit einer Resistenz gegenüber einem Pyrimidinanalogen mit einer Restriktionsenzymkarte von Fig. 1 enthält und das aus Chromosomen-DNA des Mikroorganismus *Bacillus subtilis* AA47 (IFO 14705, FERM BP 2183) erhalten wird, um dadurch die Bildung und Anreicherung von Uracil in der Kulturbrühe zu bewirken, und das Gewinnen des Uracils aus der Kulturbrühe umfaßt.

8. Verfahren wie in Anspruch 7 beansprucht, bei welchem der transformierte Mikroorganismus *Escherichia coli* TFA17 (IFO 14704, FERM BP-2182) ist.

9. Verfahren wie in Anspruch 7 beansprucht, bei welchem der transformierte Mikroorganismus *Bacillus subtilis* TFBA16 (IFO 14707, FERM BP-2184) ist.

10. Verfahren wie in Anspruch 7 beansprucht, bei welchem der transformierte Mikroorganismus *Bacillus subtilis* TFAA47 (IFO 14708, FERM BP-2185) ist.

## Revendications (Revendications pour l'(les) Etat(s) contractant(s) suivant(s): AT, BE, CH, DE, FR, GB, GR, IT, LI, LU, NL, SE)

1. ADN comportant une région qui constitue un gène de résistance à un analogue de la pyrimidine, ledit ADN présentant la carte d'enzymes de restriction de la Figure 1 et étant obtenu à partir d'un ADN chromosomique d'un microorganisme Bacillus subtilis AA47 (IFO 14705, FERM BP-2183).

2. Plasmide dans lequel est incorporé un ADN conforme à la revendication 1.

3. Culture pure d'un microorganisme du genre Escherichia ou Bacillus, transformé avec un plasmide conforme à la revendication 2.

4. Culture pure conforme à la revendication 3, qui est Escherichia coli TFA17 (IFO 14704, FERM BP-2182).

5. Culture pure conforme à la revendication 3, qui est Bacillus subtilis TFBA16 (IFO 14707, FERM BP-2184).

6. Culture pure conforme à la revendication 3, qui est Bacillus subtilis TFAA47 (IFO 14708, FERM BP-2185).

7. Procédé de production de l'uracile, qui comporte le fait de cultiver, dans un milieu de culture approprié, un microorganisme du genre Escherichia ou Bacillus, transformé avec un plasmide dans lequel est incorporé un gène de résistance à un analogue de la pyrimidine, présentant la carte d'enzymes de restriction de la Figure 1 et obtenu à partir d'un ADN chromosomique d'un microorganisme Bacillus subtilis AA47 (IFO 14705, FERM BP-2183), pour provoquer ainsi la formation et l'accumulation d'uracile dans le bouillon de culture, et le fait de récupérer l'uracile à partir de ce bouillon.

8. Procédé conforme à la revendication 7, dans lequel le microorganisme transformé est Escherichia coli TFA17 (IFO 14704, FERM BP-2182).

9. Procédé conforme à la revendication 7, dans lequel le microorganisme transformé est Bacillus subtilis TFBA16 (IFO 14707, FERM BP-2184).

10. Procédé conforme à la revendication 7, dans lequel le microorganisme transformé est Bacillus subtilis TFAA47 (IFO 14708, FERM BP-2185).

## Revendications (Revendications pour l'(les) Etat(s) contractant(s) suivant(s): ES)

1. Procédé de préparation d'un ADN comportant une région qui constitue un gène de résistance à un analogue de la pyrimidine, ledit ADN présentant la carte d'enzymes de restriction de la Figure 1 et étant obtenu à partir d'un ADN chromosomique d'un microorganisme Bacillus subtilis AA47 (IFO 14705, FERM BP-2183), lequel procédé comporte l'extraction de l'ADN chromosomique du microorganisme donneur.

2. Procédé de préparation d'un plasmide dans lequel est incorporé un ADN préparé conformément à la revendication 1, lequel procédé comporte l'insertion de l'ADN mentionné ci-dessus dans un ADN vecteur.

3. Procédé de préparation d'une culture pure d'un microorganisme du genre Escherichia ou Bacillus, capable de produire de l'uracile, lequel procédé comporte la transformation d'un microorganisme récepteur avec un plasmide préparé conformément à la revendication 2.

4. Procédé conforme à la revendication 3, dans lequel on prépare une culture pure d'un microorganisme qui est Escherichia coli TFA17 (IFO 14704, FERM BP-2182).

5. Procédé conforme à la revendication 3, dans lequel on prépare une culture pure d'un microorganisme qui est Bacillus subtilis TFBA16 (IFO 14707, FERM BP-2184).

6. Procédé conforme à la revendication 3, dans lequel on prépare une culture pure d'un microorganisme qui est Bacillus subtilis TFAA47 (IFO 14708, FERM BP-2185).

7. Procédé de production de l'uracile, qui comporte le fait de cultiver, dans un milieu de culture approprié, un microorganisme du genre Escherichia ou Bacillus, transformé avec un plasmide dans lequel est incorporé un gène de résistance à un analogue de la pyrimidine, présentant la carte d'enzymes de restriction de la Figure 1 et obtenu à partir d'un ADN chromosomique d'un microorganisme Bacillus subtilis AA47 (IFO 14705, FERM BP-2183), pour provoquer ainsi la formation et l'accumulation d'uracile dans le bouillon de culture, et le fait de récupérer l'uracile à partir de ce bouillon.

8. Procédé conforme à la revendication 7, dans lequel le microorganisme transformé est Escherichia coli TFA17 (IFO 14704, FERM BP-2182).

9. Procédé conforme à la revendication 7, dans lequel le microorganisme transformé est Bacillus subtilis TFBA16 (IFO 14707, FERM BP-2184).

10. Procédé conforme à la revendication 7, dans lequel le microorganisme transformé est Bacillus subtilis TFAA47 (IFO 14708, FERM BP-2185).
